# EUROPÄISCHE PATENTSCHRIFT

(11) **EP 2 526 900 B1**
(45) Veröffentlichungstag und Bekanntmachung des Hinweises auf die Patenterteilung: **07.02.2018**
(21) Anmeldenummer: 12003972.2
(22) Anmeldetag: 22.05.2012
(51) Int. Cl.: A61F 2/30, A61F 2/38

(54) **Form zur Herstellung einer Femurkomponente eines Kniegelenkspacers**
Mould for producing a femur component of a knee joint spacer
Moule destiné à la fabrication d'un composant de fémur d'un espaceur d'articulation du genou

(30) Priorität: 24.05.2011 DE 102011103005
(43) Veröffentlichungstag der Anmeldung: 28.11.2012
(73) Patentinhaber: Heraeus Medical GmbH, 61273 Wehrheim (DE)
(72) Erfinder: Wüst, Edgar, 64823 Groß-Umstadt (DE)
(74) Vertreter: Heraeus IP

(56) Entgegenhaltungen:
- US-A1- 2005 107 885
- US-A1- 2010 102 484

## Beschreibung

Die vorliegende Erfindung betrifft eine Form zur Herstellung einer Femurkomponente eines Kniegelenkspacers, sie betrifft insbesondere eine mehrteilige Form.

In Deutschland werden zurzeit ca. 150.000 Knietotalendoprothesen pro Jahr implantiert. Als relevante Komplikationen können Früh- und Spätinfektionen des Implantatlagers auftreten. Kommt es zur Infektion im Bereich des künstlichen Gelenkes, wird zunächst versucht, den Infekt mittels konsequenter chirurgischer Sanierung kombiniert mit testgerechten Antibiotika zu behandeln. In sehr vielen Fällen ist dies alleine jedoch nicht wirksam. Die infizierte Prothese muss dann operativ entfernt werden. Dabei wird gleichzeitig der durch die Infektion kompromittierte Knochen mit entfernt.

Nachdem die infizierte Prothese entfernt wurde, platziert man, um die Stabilität des Gelenkes aufrecht zu erhalten, an Stelle der Prothese einen sogenannten Platzhalter oder Spacer.

Die zurzeit auf dem Markt befindlichen Spacer bestehen aus Kunststoff (PMMA). Die Geometrie der Kniegelenkspacer orientiert sich im Groben am Design einer Primärprothese. Unberücksichtigt bleiben dabei die individuellen Zuschnitte am Femurknochen, die von der ursprünglich implantierten Prothese vorgegeben sind. Die bisherigen verfügbaren Spacer können nicht wieder aufbereitet werden, das heißt sie dienen zum Einmalgebrauch.

Alternativ zu den oben genannten Spacer verwenden manche Operateure Platzhalter, die während der Operation eigenhändig aus Knochenzement hergestellt werden, die wahlweise antibiotisch wirksame Bestandteile enthalten können. Dabei handelt es sich um Provisorien, zu deren Herstellung werden verwendet werden.

Spacer oder Platzhaltervorrichtungen für das Kniegelenk weisen einen ZweiKomponenten-Aufbau auf, wobei eine Femurkomponente (verbunden mit dem Oberschenkelknochen) und eine Tibiakomponente (verbunden mit dem Schienbeinplateau) zu einem derartigen Kniegelenkspacer gehören.

So wird zum Beispiel in EP 1 082 199 B1 eine Zementform für ein provisorisches Implantat beschrieben, bei dem es sich unter anderem auch um die Femurkomponente eines Kniegelenkspacers handelt. Die Form ist aus zwei spiegelbildlichen Teilformen aufgebaut. Sie besteht aus einem zerreißbaren, flexiblen Silikonmaterial und weist ferner einen Entfernungsmechanismus zum Auseinanderreißen der beiden Teilformen auf. Durch das elastische Material können die Teilformen von dem provisorischen Implantat abgeschält werden.

Ein Nachteil dieser Form besteht darin, dass sie elastisch ist, und demzufolge der Spacer nicht immer exakt ausgeformt wird. Ein Nacharbeiten ist häufig notwendig. In dem Dokument US20100102484 ist eine relevante Form für eine Femurkomponente gezeigt, die Verschlusselemente aufweist. Eine Aufgabe der vorliegenden Erfindung besteht daher darin, eine Form für die Femurkomponente eines Kniegelenkspacers bereitzustellen, mit deren Hilfe Femurkomponenten hergestellt werden können, die gar nicht oder nur geringfügig nachgearbeitet werden müssen.

Diese Aufgabe wird durch den Gegenstand mit den Merkmalen des Anspruchs 1 gelöst. Weiterbildungen der Erfindung sind durch die Unteransprüche definiert.

Erfindungsgemäß weist die Form für die Femurkomponente eines Kniegelenkspacers eine erste Teilform zur Ausbildung eines tibiaseitigen Bereichs mit einer gerundeten, tibiaseitigen Oberfläche für ein Zusammenwirken mit einer Tibiakomponente des Kniegelenkspacers und eine zweite Teilform zur Ausbildung eines femurseitigen Bereichs mit femurseitigen Oberflächen zur Aufnahme des Oberschenkelknochen, auf.

Diese Art der Form macht es möglich, die Form auseinander nehmen zu können, ohne dass sie elastisch verformt wird. Die Teilformen werden einfach auseinandergezogen, ohne sie zu verdrehen oder zu verbiegen. Bevorzugt besteht die Form ausschließlich aus diesen zwei Teilformen. Dies hat zum einen Vorteile im Hinblick auf den Herstellungsprozess, da lediglich zwei Teile gefertigt werden müssen. Zum anderen gibt es Vorteile im Hinblick auf die Handhabung, da selbst in der angespannten Situation einer Operation beim Zusammensetzen und Auseinandernehmen der Form kaum Fehler auftreten können.

Bevorzugt sind die beiden Teilformen an ihrer Grenzfläche nicht eben, sondern weisen Vorsprünge und Vertiefungen oder Stufen auf, die ein Verrutschen der Teilformen gegeneinander verhindern.

Die Teilformen sind über lösbare Verschlusselemente aneinander verbindbar. Dies geschieht über Rasteinrichtungen, diese Verschlusselemente sind integriert mit den Teilformen ausgebildet.

Beispielsweise können Rastelemente an einer Teilform in komplementäre Verbindungselemente oder Vertiefungen der anderen Teilform greifen. Besonderes bevorzugt sind die Rastelemente oder als Laschen ausgebildet, die an einer Teilform befestigt sind. Bevorzugt handelt es sich bei den Rastelementen um Vorsprünge, wie zum Beispiel Haken. Bei den Verbindungselementen der anderen Teilform kann es sich zum Beispiel um Rillen handeln, in die die Haken passen. Durch ihre längliche Ausbildung besitzen die Laschen eine gewisse Elastizität, die es ermöglicht, dass sie über die zweite Teilform geschoben werden können. Diese Elastizität kann jedoch auch durch die Verwendung eines elastischen Materials für die Laschen bereitgestellt werden.

Zur Herstellung der Femurkomponente eines Kniegelenkspacers wird zunächst ein Knochenzementteig mit den notwendigen Antibiotika hergestellt. Dieser Knochenteig wird über eine Einfüllöffnung in einer der beiden Teilformen in die Form eingefüllt. Die Einfüllöffnung kann an einer beliebigen Stelle angeordnet sein. Bevorzugt ist die Einfüllöffnung mittig in der Teilform angeordnet, die die Femurseite des Formkörpers ausbildet. Es ist aber auch denkbar, dass sie in die Teilform eingelassen ist,
Die Formen können aus jedem beliebigen biokompatiblen Material mit ausreichender Festigkeit hergestellt werden. Es gibt verschiedene Polymere, die für diese Anwendungen geeignet sind. Besonders geeignet sind formstabile, harte Materialien. Beispiele hierfür sind Polyethylen, Polypropylen, Polyamid, Polyetherketon, Teflon und Polyoxymethylen. Besonders geeignet sind glaskugelverstärkte thermoplastische Kunststoffe, wie glaskugelverstärktes Polyethylen, glaskugelverstärktes Polypropylen, glaskugelverstärktes Polyamid. Diese Materialien haben eine ausreichende Festigkeit, so dass sie sich während der Herstellung der Spacer nicht verformen.

Prinzipiell sind alle biokompatiblen Materialien geeignet, aus denen sich die Formen herstellen lassen, unter der Voraussetzung, dass sie keine chemische Reaktion mit Knochenzement oder Antibiotikum eingehen. Denkbar sind somit auch Metalle, wie z.B. Edelmetall oder teflonbeschichtetes Aluminium.

Erfindungsgemäß bedeutet "formstabil", dass sich die Form während des Füllvorgangs nicht verzieht, so dass die Femurkomponente exakt ausgeformt wird und mit der Tibiakomponente eine formschlüssige Gleitpaarung bildet, ohne dass ein Nacharbeiten notwendig wird.

Im Folgenden werden bevorzugte Ausführungsformen der vorliegenden Erfindung anhand der beiliegenden Zeichnungen genauer beschrieben. Es zeigen:
Fig. 1 eine perspektivische Darstellung einer bevorzugten Ausführungsform einer erfindungsgemäßen Form,
Fig. 2 eine perspektivische Ansicht einer ersten Teilform der Form nach Fig. 1,
Fig. 3 eine perspektivische Ansicht einer zweiten Teilform der Form nach Fig. 1 mit Femurkomponente,
Fig. 4 ein Beispiel in perspektivischer Darstellung,
Fig. 5 ein weiteres Beispiel in perspektivischer Darstellung in geschlossenem Zustand,
Fig. 6 das weitere Beispiel perspektivischer Darstellung in geöffnetem Zustand, und
Fig. 7 eine Femurkomponente nach Entnahme aus einer Form.

In den Figuren bezeichnen dieselben Bezugszeichen gleiche oder funktionsgleiche Komponenten, soweit nichts Gegenteiliges angegeben ist.

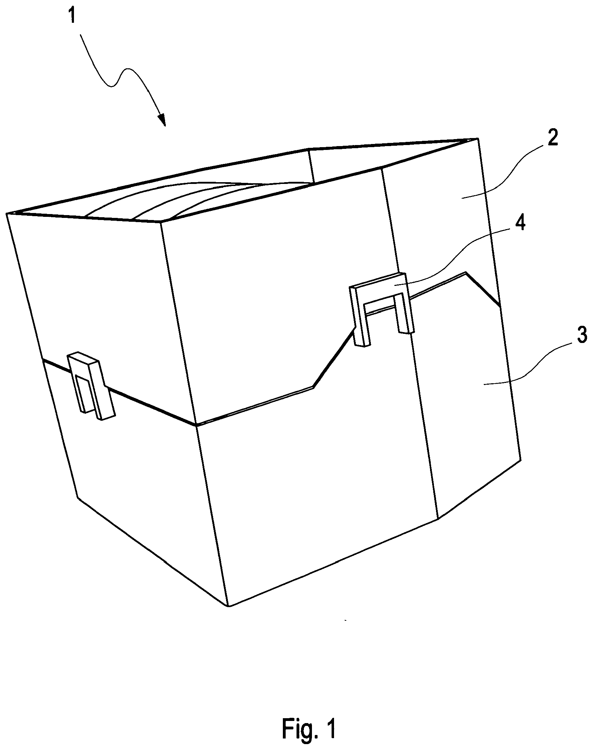

Fig. 1 zeigt eine perspektivische Darstellung eines Ausführungsbeispiels einer erfindungsgemäßen Form 1 zur Herstellung der Femurkomponente eines Kniegelenkspacers. Die Gestalt (Innenkontur) der Form 1 richtet sich nach dem Implantat, welches die Femurkomponente für eine gewisse Zeit ersetzen soll. Die Form 1 weist in diesem Ausführungsbeispiel zwei Teilformen 2,3, nämlich eine tibiaseitige Teilform 2 und eine femurseitige Teilform 3 auf. Diese Ausführungsform weist nach außen hin glatte Wände auf, die nach Zusammensetzen der beiden Teilformen 2,3 ausgebildet werden. Um die Teilformen aneinander zu fixieren, werden Verschlusseinrichtungen 4 verwendet.

Die zusammengesetzten Teilformen 2,3, die gemeinsam die Form 1 bilden, bilden mit ihren aneinandergrenzenden Außenseiten bevorzugt eine glatte Fläche. An dieser glatten Fläche können auf einfache Weise Verschlusselemente angebracht werden. In der bevorzugten Ausführungsform handelt es sich bei diesen Verschlusselementen um Haken, die an der zweiten Teilform 3 an Laschen 4 ausgebildet sind. Diese greifen in Vertiefungen an der ersten Teilform 2. Indem die Laschen 4 angehoben und die Teilformen 2 und 3 auseinandergezogen werden, können die Teilformen 2 und 3 einfach voneinander gelöst werden.

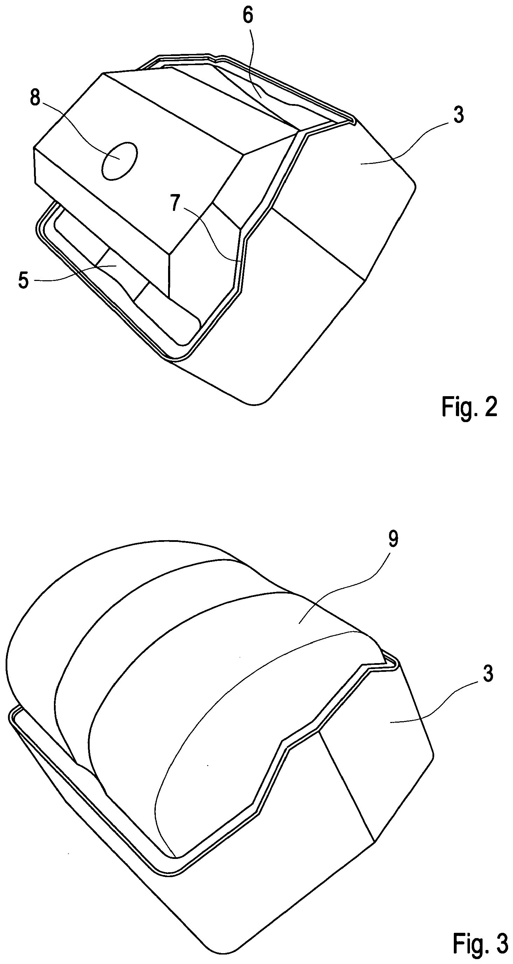

In Fig. 2 ist die Teilform 3 dargestellt, welche die femurseitige Oberfläche des Spacers ausbildet. Die Verschlusselemente sind aus Gründen der Übersichtlichkeit weggelassen worden. Die Teilform 3 weist eine Einfüllöffnung 8 auf, durch die die Zementmasse in die Form eingefüllt wird. An den Außenseiten sind zwei Vertiefungen 5,6 ausgebildet, in denen der äußere Kondylenabschnitt und der Partellaschild des Spacers ausgeformt werden.

Die Teilform 3 weist einen Außenumfang auf, der durch eine umlaufende Wand gebildet wird, die einen sechseckigen Grundriss umgibt, dessen Fläche aus einem Rechteck und einem Trapez zusammengesetzt ist. Die umlaufende Wand weist Stufen 7 auf, derart dass sie bereichsweise unterschiedliche Höhen hat, die komplementär zu Höhen einer vergleichbaren Wand der Teilform für die Tibiaseite sind. Auf diese Weise können die Teilformen nur auf eine Art zusammengesetzt werden, so dass Fehler vermieden werden.

Fig. 3 zeigt ebenfalls die Teilform 3, hier jedoch mit Femurkomponente 9, wobei der abgerundete Bereich der Tibiaseite der Femurkomponente nach außen offen liegt.

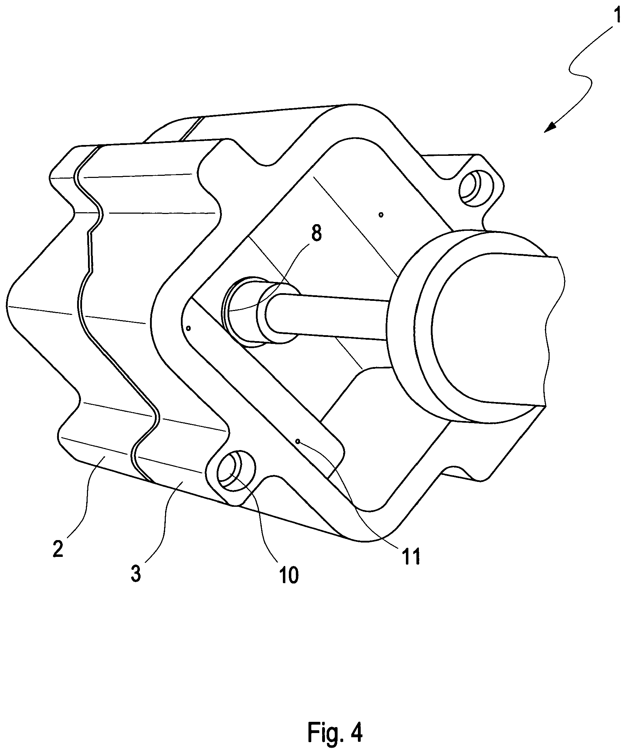

Fig. 4 zeigt ein alternatives Beispiel, das nicht unter den Schutzbereich der Erfindung fällt, in perspektivischer Darstellung, bei der die beiden Teilformen 2,3 über Schraubverbindungen 10 miteinander verbunden sind. Die Außenwand der Form 1 beschreibt hier ein Sechseck. In der Mitte jeder der vier Außenflächen ist über beide Teilformen 2,3 ein vorspringender Bereich ausgebildet. In den vorspringenden Bereich einer beiden Teilformen 2,3 ist ein Gewinde eingelassen, während in den vorspringenden Bereich der anderen Teilform 3,2 ein Loch eingelassen ist, durch das eine Schraube geführt werden kann, die in das Gewinde geschraubt werden kann.

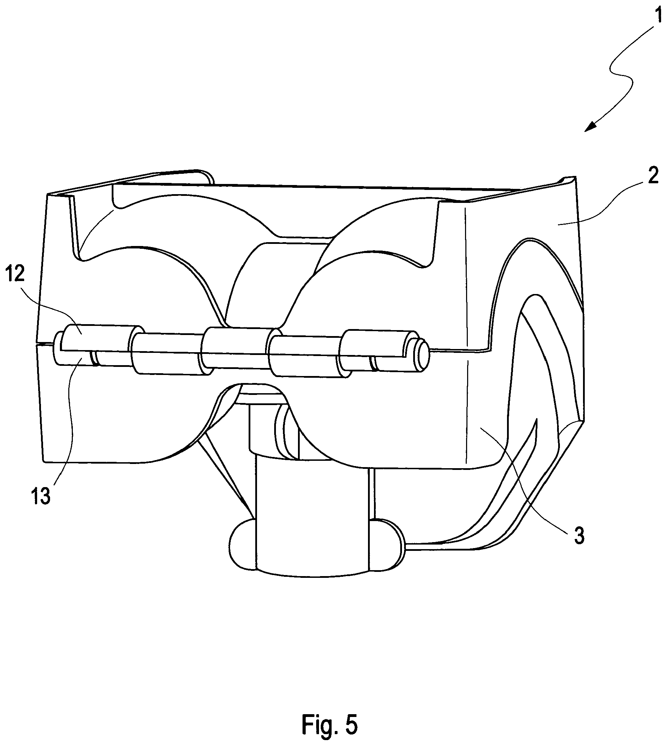

Fig. 5 zeigt weiteres alternatives Beispiel, das nicht unter den Schutzbereich der Ansprüche fällt, in perspektivischer Darstellung, bei der die beiden Teilformen 2,3 über ein Stiftlappenband miteinander verbunden sind. Die Lappen 12 des Lappenbandes müssen keine vollständigen Rohre bilden, sondern können auch nach innen offen sein. Sie werden über einen Stift 13 miteinander verbunden.

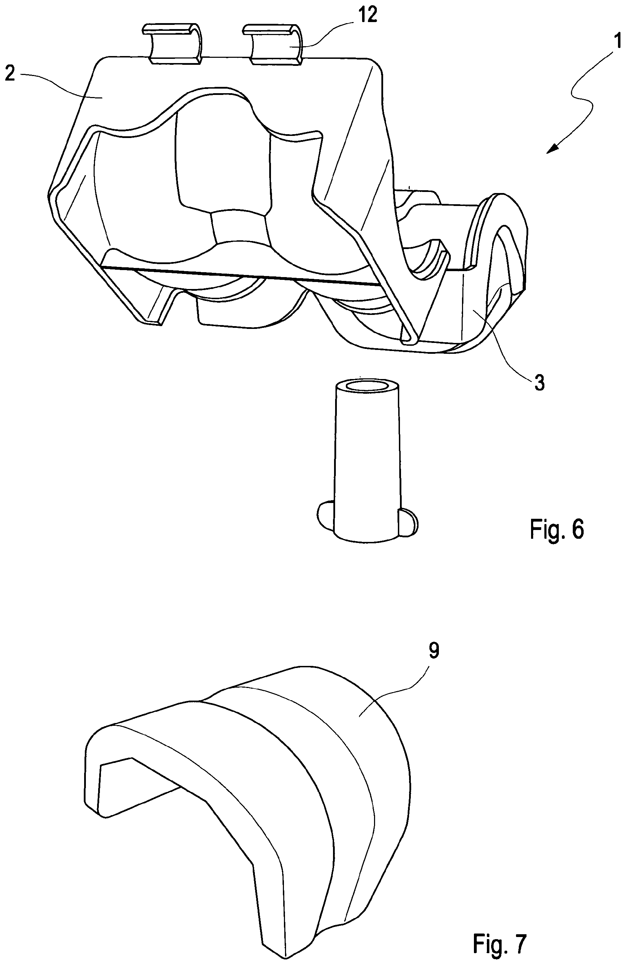

Fig. 6 zeigt die Form nach Fig. 5 in geöffnetem Zustand. Zur Entnahme des Spacers muss lediglich ein Stift 13 aus dem Band gezogen werden, um die Form aufzuklappen.

Um die Form zu füllen wird zunächst ein Knochenzementteig angerührt. Patientenspezifisch kann gleichzeitig ein oder mehrere entsprechende Antibiotika unter den Teig gerührt werden. Mit Hilfe z.B. einer Knochenzementspritze kann der Teig dann über eine Einfüllöffnung in die Form eingefüllt werden. Damit die Form vollständig gefüllt werden kann und sich keine Lufträume in der Form bilden, sind an den äußersten Hohlraumenden Entlüftungsöffnungen in die Wand der Form eingelassen. Füllt sich die Form nun nach und nach mit dem Knochenzement, so kann über diese Öffnungen die Luft entweichen.

Eine Femurkomponente nach Entnahme aus der Form ist in Fig. 7 dargestellt.

Zur Verstärkung eines festen Sitzes an dem Femurende können die femurseitigen Oberflächen zumindest teilweise mit einer bestimmten Oberflächenrauigkeit und/oder einem bestimmten Profil versehen sein.

### Bezugszeichenliste

- 1: Form
- 2: Teilform
- 3: Teilform
- 4: Laschen
- 5: Vertiefung
- 6: Vertiefung
- 7: Stufen
- 8: Einfüllöffnung
- 9: Femurkomponente
- 10: Schraubverbindungen
- 11: Entlüftungsöffnungen
- 12: Lappenband
- 13: Stift

## Patentansprüche

1. Form für die Femurkomponente eines Knie-Spacers, aufweisend zwei Teilformen (2,3), welche voneinander lösbar sind, wobei
eine erste Teilform (2) zur Ausformung der Tibiaseite der Femurkomponente und eine zweite Teilform (3) zur Ausformung der Oberschenkelknochenseite der Femurkomponente dient,
wobei beide Teilformen (2,3) über eine oder mehrere lösbare Verschlusseinrichtungen miteinander verbindbar sind,
**dadurch gekennzeichnet, dass**
es sich bei den lösbaren Verschlusseinrichtungen um Rastverschlüsse handelt, und die Verschlusselemente integriert mit den Teilformen ausgebildet sind.

2. Form nach Anspruch 1,
**dadurch gekennzeichnet, dass**
die erste Teilform (2) und die zweite Teilform (3) vollständig voneinander lösbar sind.

3. Form nach einem der Ansprüche 1 oder 2,
**dadurch gekennzeichnet, dass**
der Rastverschluss einen Haken aufweist, der an einer Lasche (4) ausgebildet ist, welche an einer der Teilformen (2,3) befestigt ist, und eine Vertiefung aufweist, die in der anderen Teilform (3,2) derart ausgebildet ist, dass der Haken in diese einrasten kann.

4. Form nach einem der vorhergehenden Ansprüche,
**dadurch gekennzeichnet, dass**
die beiden Teilformen (2,3) an ihren aneinanderstoßenden Grenzflächen komplementär zueinander ausgebildete vorspringende und zurückweichende Bereiche (7) aufweisen, derart dass ein Verschieben der Teilformen gegeneinander verhindert wird.

5. Form nach einem der vorhergehenden Ansprüche,
**dadurch gekennzeichnet, dass**
die Form (1) eine umlaufende Wand mit ebenen Wandbereichen aufweist.

6. Form nach einem der vorhergehenden Ansprüche,
**dadurch gekennzeichnet, dass**
die Form (1) aus einem formstabilen Material gefertigt ist.

7. Form nach einem der vorhergehenden Ansprüche,
**dadurch gekennzeichnet, dass**
es sich bei dem Material um einen Kunststoff handelt.

8. Form nach Anspruch 7,
**dadurch gekennzeichnet, dass**
der Kunststoff aus der Gruppe ausgewählt ist, die aus Polypropylen, Polyethylen und Polyamid besteht.

9. Form nach Anspruch 8,
**dadurch gekennzeichnet, dass**
der Kunststoff ein glaskugelverstärktes Polypropylen oder Polyamid ist.

## Claims

1. Mould for the femoral component of a knee spacer, comprising two sub-moulds (2, 3), which can be detached from each other, whereby
a first sub-mould (2) serves for forming the tibial side of the femoral component and a second sub-mould (3) serves for forming the femoral bone side of the femoral component,
whereby
both sub-moulds (2, 3) can be connected to each other by means of one or more detachable locking facilities,
**characterised in that**
the detachable locking facilities are snap-in locks and the locking elements are provided such as to be integrated in the sub-moulds.

2. Mould according to claim 1,
**characterised in that**
the first sub-mould (2) and the second sub-mould (3) can be completely detached from each other.

3. Mould according to any one of the claims 1 or 2,
**characterised in that**
the snap-in lock comprises a hook that is provided on a lug (4), which is attached to one of the sub-moulds (2, 3), and comprises a recess, which is provided appropriately in the other sub-mould (3, 2) such that the hook can snap into it.

4. Mould according to any one of the preceding claims,
**characterised in that**
the two sub-moulds (2, 3) comprise, on their adjacent boundaries, projecting and receding regions (7) that are provided to be complementary to each other, such that any shifting of the sub-moulds with respect to each other is prevented.

5. Mould according to any one of the preceding claims,
**characterised in that**
the mould (1) comprises a circumferential wall with planar wall regions.

6. Mould according to any one of the preceding claims,
**characterised in that**
the mould (1) is manufactured from a shape-stable material.

7. Mould according to any one of the preceding claims,
**characterised in that**
the material is a plastic material.

8. Mould according to claim 7,
**characterised in that**
the plastic material is selected from the group consisting of polypropylene, polyethylene, and polyamide.

9. Mould according to claim 8,
**characterised in that**
the plastic material is a glass bead-reinforced polypropylene or polyamide.

## Revendications

1. Moule pour le composant fémoral d'un écarteur de genou, présentant deux moules partiels (2, 3) qui peuvent être détachés l'un de l'autre, dans lequel un premier moule partiel (2) sert au démoulage du côté tibial du composant fémoral et un second moule partiel (3) sert au démoulage du côté osseux de cuisse du composant fémoral,
dans lequel
les deux moules partiels (2, 3) peuvent être connectés l'un à l'autre par le biais d'un ou plusieurs dispositifs de fermeture amovibles,
**caractérisé en ce que**
il s'agit pour les dispositifs de fermeture amovibles de fermetures par encliquetage et les éléments de fermeture sont réalisés de manière intégrée aux moules partiels.

2. Moule selon la revendication 1,
**caractérisé en ce que**
le premier moule partiel (2) et le second moule partiel (3) peuvent être détachés entièrement l'un de l'autre.

3. Moule selon une des revendications 1 ou 2,
**caractérisé en ce que**
la fermeture par encliquetage présente un crochet qui est réalisé sur une attache (4) qui est fixée à un des moules partiels (2, 3), et présente un renfoncement qui est réalisé dans l'autre moule partiel (3, 2) de telle sorte que le crochet peut s'encliqueter dans celui-ci.

4. Moule selon une des revendications précédentes,
**caractérisé en ce que**
les deux moules partiels (2, 3) présentent des régions en saillie et en retrait (7) réalisées de manière complémentaire les unes aux autres sur leurs faces limites contiguës de telle sorte qu'un coulissement des moules partiels l'un contre l'autre est empêché.

5. Moule selon une des revendications précédentes,
**caractérisé en ce que**
le moule (1) présente une paroi périphérique avec des régions de paroi planes.

6. Moule selon une des revendications précédentes,
**caractérisé en ce que**
le moule (1) est fabriqué en un matériau indéformable.

7. Moule selon une des revendications précédentes,
**caractérisé en ce que**
il s'agit pour le matériau d'un plastique.

8. Moule selon la revendication 7,
**caractérisé en ce que**
le plastique est sélectionné parmi le groupe qui se compose du polypropylène, polyéthylène et polyamide.

9. Moule selon la revendication 8,
**caractérisé en ce que**
le plastique est un polypropylène ou polyamide renforcé par des billes de verre.
